# EUROPEAN PATENT SPECIFICATION

(11) **EP 0 662 135 B1**
(45) Date of publication and mention of the grant of the patent: **23.07.1997**
(21) Application number: 93921022.5
(22) Date of filing: 28.09.1993
(51) Int. Cl.: C12N 15/56, C12N 9/36, C12Q 1/68, C07K 14/00

(54) **ALTERATION OF POLYPEPTIDES**
VERÄNDERUNG VON POLYPEPTIDEN
MODIFICATION DE POLYPEPTIDES

(30) Priority: 28.09.1992 GB 9220418
(43) Date of publication of application: 12.07.1995
(73) Proprietor: UNILEVER PLC, London EC4P 4BQ (GB); UNILEVER N.V., 3013 AL Rotterdam (NL)
(72) Inventor: VARVILL, Katherine, West Sussex P020 8AN (GB); PICKERSGILL, Richard, William, Farnham, Surrey GU10 3BH (GB); GOULD, Grahame, Warwich, Bedfordshire MK41 7AA (GB); GOODENOUGH, Peter, William, Reading, Berkshire RG1 5RD (GB); MOSELY, Bevan, Edwin, Bowen, Berkshire RG1 5RD (GB)
(74) Representative: van der Toorren, Johannes, Drs.
(86) International application number: GB9302026
(87) International publication number: WO9408018

(56) References cited:
- EP-A- 0 242 836
- WO-A-91/04270
- BIOCHEMISTRY vol. 30, no. 15 , 1991 , EASTON, PA US pages 3621 - 3627 POOLE, L. ET AL. 'Deletion of the Omega-loop in the active site of staphylococcal nuclease. 1. Effect on catalysis and stability' cited in the application
- GENE vol. 96 , 1990 , AMSTERDAM NL pages 125 - 128 LANDT, O. ET AL. 'A general method for rapid site-directed mutagenesis using the polymerase chain reaction'
- SCIENCE vol. 244 , 7 April 1989 , LANCASTER, PA US pages 82 - 85 KUIPERS, O. ET AL. 'Enhanced activity and altered specificity of phospholipase A2 by deletion of a surface loop' cited in the application

## Description

### Field of the Invention

This invention relates to altered polypeptides and a method of obtaining altered polypeptides.

### Background of the Invention

Improvements in the understanding of protein structure and in recombinant DNA techniques have enabled attempts to alter the primary structure of polypeptides by manipulating the structural genes encoding them. Typically such manipulation may be performed by site-directed mutagenesis (SDM). Alteration of the primary structure will often result in changes, to a variable extent, in the secondary, tertiary, or even quarternary structure of a protein with concomitant effects on the biological and/or physico-chemical behaviour of the protein. Thus protein engineering in this way offers a means of adapting the properties of a protein, such as an enzyme, for a particular purpose. Despite these increases in knowledge and skills, it remains difficult to predict generally how the characteristics of a protein will be altered by particular changes to its structure.

One principle of assistance in research of this type is the intuitively obvious and empirically observed guideline that making changes to regions of polypeptide structure conserved during evolution, or regions which are not obviously variable from the comparison of homologous polypeptides, will normally have profoundly unfavourable effects on the biological activity of the molecule. A second guideline, which is equally intuitively obvious to those skilled in the art, is that the larger the region of structure altered, the greater the possibility of destroying a contact or interaction essential for biological activity.

A limited number of studies are described in the prior art where attempts have been made to alter the primary structure of enzymes by altering more than one or two consecutive amino acid residues. For example Kuipers et al. (1989, Science 244, 82-85) disclosed a mutant form of the enzyme porcine pancreatic phospholipase A₂ (PLA₂), in which 5 amino acids were removed. In addition to this straightforward deletion, these workers also made amino acid substitutions at other sites to "maintain maximal sequence homology with elapid venom PLA₂s in the region of the loop". That is, amino acid substitutions were made at other sites, using the known sequences of the closely related PLA₂s (found in some snake venoms) as a model.

Poole et al., (1991, Biochemistry 30, 3621-3627) disclosed a random mutation of staphylococcal nuclease in which residues 44-49 were deleted. Further work on the same enzyme (Flanagan et al., 1992, PNAS 89, 748-752) revealed that a truncated form, lacking 13 amino acids at the carboxyl terminal, retained some enzyme activity.

Kuchinke (1989, Biochem. Biophys. Res. Comm 159, 927-932) described the results of work involving the expression of exon 2 of a lysozyme gene. The expressed polypeptide comprised residues 28-81 of the enzyme (which include the catalytic residues and some of the carbohydrate binding residues). However this fragment exhibited only 1/40,000th of the activity of the native enzyme and so could not be considered as biologically functional.

Thus most of the work reported so far concerns simply deletions of certain stretches of amino acids or, in the case of Kuipers et al., a deletion accompanied by substitutions at other sites so as to follow as closely as possible the sequence of enzymes known to be closely homologous to the molecule of interest.

### Summary of the Invention

In one aspect the invention provides a biologically functional altered lysozyme molecule, comprising fewer amino acids and being reduced in size compared with an unaltered lysozyme molecule, wherein the altered lysozyme lacks at least part of the loop region of amino acid residues Cysteine 64 to Cysteine 80 of the unaltered molecule.

The overall external dimensions of the altered polypeptide molecule are smaller ("reduced in size") compared with the unaltered polypeptide.

Preferably the at least part of the loop region is replaced with a sequence containing fewer amino acids, said sequence being different to any sequence present in the replaced part of the unaltered molecule.

A biologically functional polypeptide is intended to mean any amino acid sequence having an activity which can exert an effect on biological systems, such activity including reaction catalysis, signal transduction and ligand activity (i.e. binding to another molecule). This activity is preserved in the altered polypeptide. Preferably the activity is wholly or substantially (i.e. preferably at least 10%) preserved. However, by using high concentrations of purified altered polypeptides for example, which may be obtainable by the use of recombinant DNA technology, even altered polypeptides with greatly reduced activity (e.g. down to 0.1% of the activity of the wild type polypeptide) may be useful. However, polypeptides with an activity of less than 0.1% of the wild type polypeptide are not considered as being biologically functional for the purposes of the present application. Many polypeptides have a plurality of biological functions. Clearly, depending on the desired end, it may not be necessary to retain all of these functions in the altered polypeptide. Indeed, it may be advantageous to abolish one or more of these functions.

The amino acid sequence which is lacking in the altered polypeptide comprises at least part of a loop region. The term "loop" is well known to those skilled in the art and is intended to indicate regions of a polypeptide comprising three or more amino acids in which the main chain Cₐₗₚₕₐ backbone of the molecule forms an arc or substantially U-shaped configuration or any other region connecting regular secondary structures. Typically the loop is an "omega" loop (i.e. one which is narrower at its base than at the tip). These may be stabilised by disulphide bridges.

It is a surprising feature of the present invention that it allows for the modification of regions of lysozyme molecules which have been conserved during evolution or regions which are not highly variable, without destroying the biological functional activity of the polypeptide. Thus omega and other loops which are typically highly conser may be substituted with smaller loops without entirely disrupting the overall structure and function of the molecule.

The phrase "regions conserved during evolution" is intended to refer to those structural elements which ar found to be present in all proteins having a particular defined activity and belonging to the same structural class (e.g. all 'C' type lysozymes).

A hypervariable sequence is one which may vary greatly in length and/or composition among proteins belonging to the same structural class.

The altered polypeptide is an enzyme, thus its biological activity is a catalytic function.

It is another preferred feature of the present invention that the amino acid sequence used to replace the amino acid sequence of the unaltered polypeptide is selected de novo from theoretical considerations. (Thus the present invention does not extend to the mere substitution of a region with a sequence known [or suspected] to form the same or a similar configuration in another region of the molecule or in other molecules.)

The invention may have particular advantages in allowing the altered enzyme to pass through pores which would normally exclude the unaltered enzyme. Alternatively, an advantage might lie in the increased rate of diffusion of the enzyme or in its altered structure rendering it, for example, more resistant to proteases or inhibitors or increasing or reducing its affinity for other molecules with which it may interact (e.g. receptor molecules, enzyme substrates).

In particular, the altered molecule of lysozyme having a smaller size than the unaltered molecule, may be able to penetrate bacterial spore or cell envelopes with greater ease. This can enable the altered enzyme to be more effective against those organisms upon which the unaltered molecule has some effect and, additionally, can enable the altered enzyme to be effective against certain other organisms upon which the unaltered molecule has no effect.

This is particularly useful in the case of lysozyme, as a reduced size lysozyme may find application, inter alia, in the processing of foods so as to prevent spoilage and/or contamination by micro-organisms.

The particular altered polypeptide disclosed herein is lysozyme. In particular, the invention relates to hen egg white lysozyme (HEL), although the region selected for substitution is highly conserved and thus found in the lysozyme molecules from many other sources, which are well known to those skilled in the art.

In a particular embodiment the invention provides an altered molecule of HEL, in which residues Cys 64 to Cys 80 exclusive have been replaced by the sequence asparagine-glycine-serine-asparagine. More specifically the invention provides the molecule defined above further comprising the amino acid threonine at position 94.

In another aspect, the invention provides a method of producing a biologically functional altered lysozyme molecule comprising fewer amino acids and being reduced in size compared with an unaltered lysozyme molecule comprising: altering the nucleic acid sequence encoding a biologically functional lysozyme molecule such that the latter lacks at least part of the loop region of amino acid residues Cysteine 64 to Cysteine 80 present in the unaltered lysozyme molecule; and causing expression of the altered nucleotide sequence so as to produce the biologically functional altered lysozyme.

Preferably the alteration is effected by PCR mutagenesis.

The aspects of the invention are more fully disclosed in the following illustrative examples and in the following Figures of which:
Figures 1-7 are schematic representations of genetic manipulations performed to obtain and optimise expression of wild-type and mutant lysozyme; and
Figure 8 is a bar chart showing the pH activity profile for recombinant wild-type and mutant lysozymes.

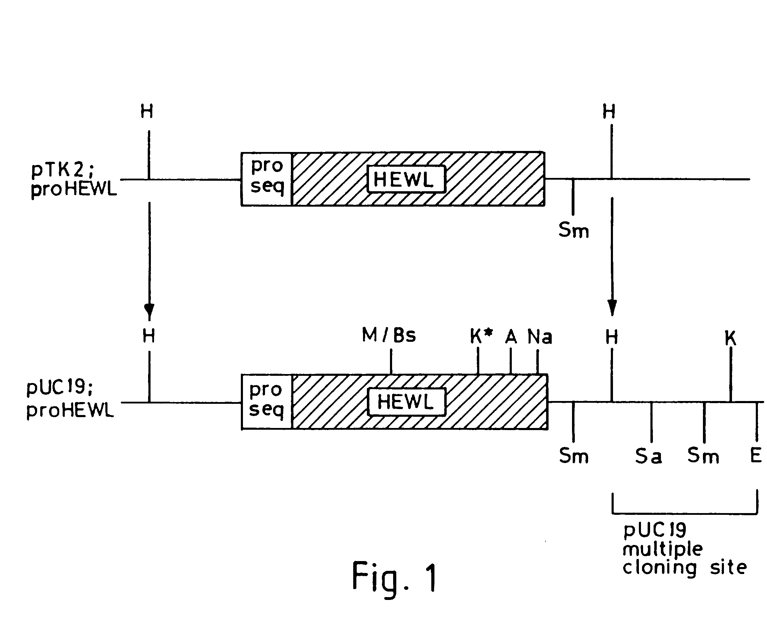

In Figures 1-7, restriction enzyme sites are identified by the following code letters: A - AatII, B - BamHI, Bs - BsaBI, E - EcoR1, H - Hind111, K - KpnI, K* - KpnI predicted from database sequence, M - MamI, Na - NaeI, Nc - NcoI, Sa - SalI, Sc - SacI, Sm - SmaI.

### Example 1

### The Cloning and Mutation of HEWL for Expression in N. crassa.

### HEWL in pUC19

A full length cDNA clone for HEWL was received into the Institute of Food Research (IFR) Reading Laboratory from IFR Norwich Laboratory as an agar stab, in the plasmid vector pTK2 (a kind gift from Dr D Archer, and described by Fischer et al., 1992 Protein Engineering 5, 593-596). This was cultured and further stabs made. However, this construct produced only very low yields of plasmid DNA by a normally efficient alkaline lysis procedure. Therefore, the clone was excised as a HindIII fragment and ligated into the vector pUC19 as shown in Fig. 1 (a schematic representation of the method by which wild-type proHEWL was transferred from pTK2 into pUC19). This is a high copy number plasmid which allowed more DNA to be obtained from a culture of XL-1 BLUE cells (stratagene) transformed with the construct. Agar stabs were made for storage.

### HEWL in M13 phage

To facilitate single-stranded DNA sequencing the HindIII clone of HEWL was isolated from pUC19;proHEWL. After ligation into HindIII-cut M13mp10, TG1 cells were transformed. This allowed recombinant plaques to be picked, from which stocks of the M13 phage containing the HEWL sequence were prepared.

Samples of these stocks were used to reinfect TG1 cells, so as to produce ssDNA for sequencing by the Sanger dideoxynucleotide procedure, as modified by Dupont Inc. for the Genesis 2000 Automatic DNA Sequencer system.

Two HEWL DNA inserts were identified by restriction enzyme analysis and then sequenced using either the M13 -20nt "universal" primer or another 24-mer primer, T12432. These allowed all bar nucleotides 222-242 of HEWL to be sequenced.

### Site directed Mutagenesis of HEWL

The first mutation undertaken was that at amino acid codon 94, which was to convert the cysteine (Cys) to a threonine (Thr) residue. This manoeuvre would prevent a mutant HEWL being produced that would retain a free cysteine residue which might affect cellular mechanisms involved in the folding of HEWL.

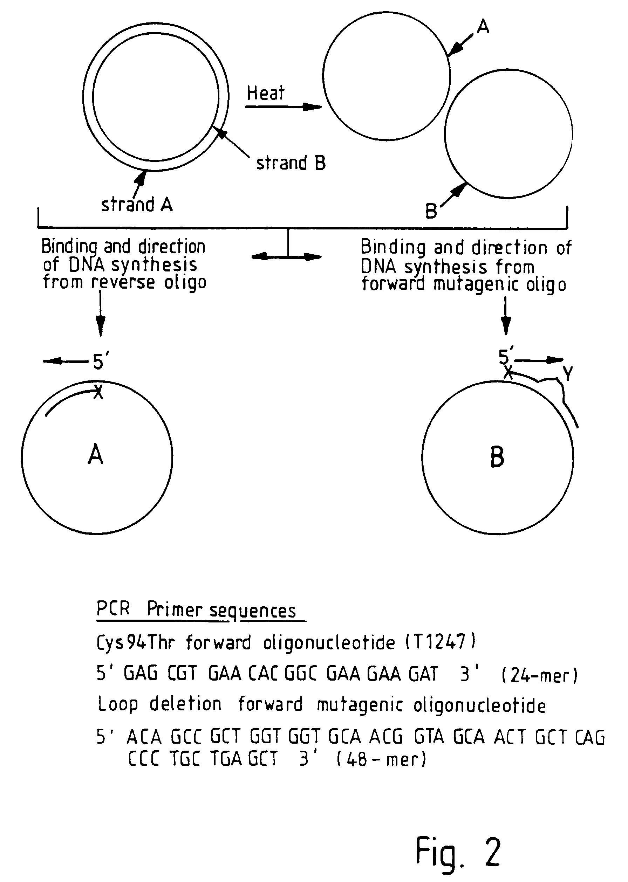

This was accomplished using the mutagenic 24-mer oligonucleotide, T1247 (Seq. ID No. 1) and the inverse PCR procedure, as depicted schematically in Fig. 2. It has been observed that this procedure is less difficult to perform and equally as reliable as the Amersham System; Version 2. It is also time saving because it does not require the subcloning steps necessary for the production of ssDNA, which is essential to the Amersham procedure.

In Figure 2, circular double-stranded plasmid DNA (consisting of strands 'A' and 'B') is denatured by heating to five single-stranded 'first cycle' templates A and B. The reverse PCR oligonucleotide binds to template A and directs DNA synthesis, whilst the forward PCR oligonucleotide binds to template B and directs DNA synthesis. On the figure, X marks point at which the forward and reverse oligonucleotides anneal "back-to-back" at their 5' ends on their respective templates. Y indicates the portion of the mutagenic primer where annealing fails to occur (due to the base mismatches) and the desired mutation is introduced.

After the first round of thermostable Taq polymerase catalysed DNA synthesis, the newly synthesized linear strands, each ending at X, allow many copies to be made, in up to 30 further rounds of amplification. Since each copy has a precisely defined end, after annealing to create double-stranded DNA, a circular plasmid can be reformed by ligation. Therefore, bacteria can be transformed, allowing mutants to be screened and expressed.

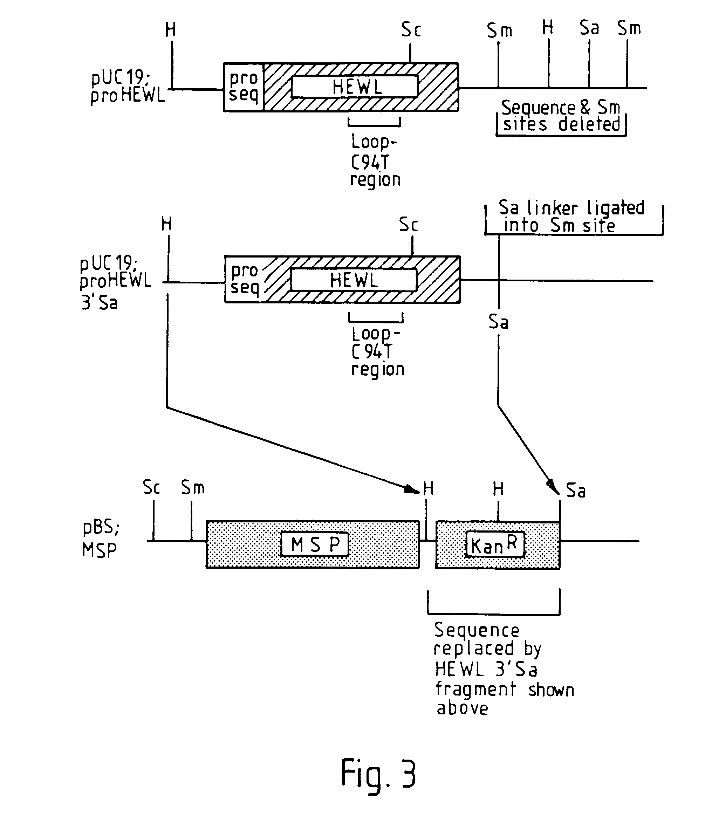

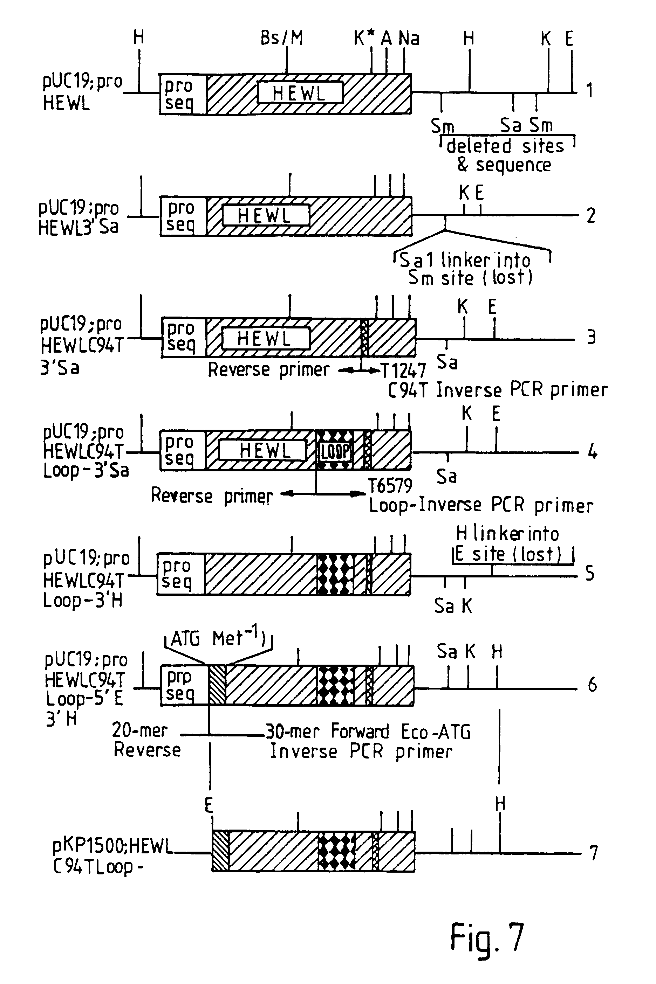

Because the plan was to insert the wild-type and mutant genes downstream of the malate synthase promoter, the inverse PCR was performed on pUC19;proHEWL which had a SalI linker inserted between the 3' SmaI sites of the plasmid, which were ablated (see Figs. 3 & 7).

Likewise, the inverse PCR procedure was successful in simultaneously carrying out the designed loop deletion and 4 codon insertion (Loop-), in the region of amino acid residues 64-80, using pUC19;proHEWLC94T 3'Sa DNA. A 48-mer oligonucleotide primer, with the sequence shown in Fig. 2, (Seq. ID No. 2) was used for this purpose.

The success of the mutations, as well as fidelity of the remaining DNA sequence for HEWL, was demonstrated by DNA sequencing using the oligonucleotide primers mentioned earlier and G13911, a 27-mer. This latter oligonucleotide is identical in sequence to a region in HEWL situated, conveniently, approximately 110 bp 5' to the 64-80 loop mutation site. The oligonucleotides used for the above mutations and sequencing, other than the M13 "Universal" primer, were designed at IFR and produced using a Dupont 300 Coder Synthesiser.

### Preparation of a plasmid vector for the transformation and expression of the HEWL gene in N. crassa.

Having achieved the desired objective of successfully mutating the wild-type HEWL, gene to create a smaller, potentially, catalytically active mutant, the next stage was to create a vector which would allow N. crassa to be transformed, with either the wild-type or the mutant gene, such that the introduced gene would be expressed under the control of an inducible promoter. It could be expected that this would allow large amounts of the two HEWL proteins to be secreted into the culture medium, alleviating problems due to isolation and renaturation of proteins from bacterial inclusion bodies.

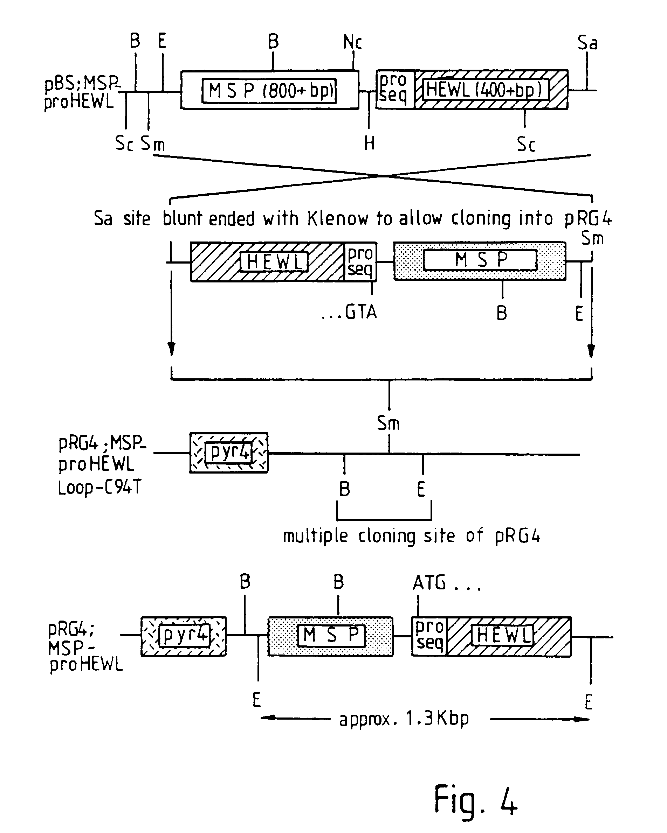

For attaining this it was planned to ligate the wild-type or mutant HEWL into a plasmid (pBs; MSP - a modified version of pBlueScript™, containing the malate synthase promoter 'MSP'), immediately downstream from the malate synthase promoter. Then this promoter-HEWL construct would be removed from the plasmid and re-introduced into another (pRG4, also derived from pBluescript™) carrying the pyr4 gene. This gene would then act as a selectable marker when N. crassa was transformed with the construct. This is reviewed in Figs. 3 & 4. Figure 3 shows schematically the manipulations used to transfer proHEWL or mutant proHEWL sequences from pUC19 to downstream of the malate synthase promoter.

The first step involved releasing the HEWL sequences (wild-type or mutant) from their pUC19 plasmid vectors. Restriction enzyme digestion with SmaI, removed the HindIII and SalI sites 3' to the HEWL insert (see Fig. 3). Then, after re-introduction of a SalI site by ligating in the 10-mer linker sequence, GGGTCGACCC, (which also ablated the SmaI sites), the HEWL gene insert could be removed as a HindIII - SalI fragment (Fig. 3).

After being recovered by electrophoresis in agarose, the fragment was ligated into the plasmid vector pBS;MSP (Fig. 3) which had similarly been HindIII and SalI digested and purified from agarose; so as to be in a form into which the HEWL HindIII-SaII fragments from pUC19;proHEWLC94TLoop- 3'Sa or pUC19;proHEWL 3'Sa could be ligated.

As Fig. 3 shows, this removed the Kan^{R} gene from pBS;MSP. Transformants were therefore selected by the release of an appropriately-sized fragment after digestion with SacI. Since the vector contains only one site for this restriction enzyme, without the HEWL insert only a linear fragment several kbp in length would be formed.

In order to transfer the MSP-HEWL construct into the pRG4 plasmid, which contained the pyr4 gene, the pBS;MSP-HEWL plasmid was restricted with SalI and SmaI. Then, after filling-in the SalI sticky end of the MSP-HEWL insert with Klenow enzyme, it was possible to blunt-end ligate the fragment into SmaI-cut pRG4, which has a unique SmaI site in the polylinker region.

The procedure is illustrated schematically in Figure 4, which shows the transfer of wild-type and mutant MSP (Malate Synthase promoter)-proHEWL sequences into pRG4, the vector used for transformation of N. crassa spheroplasts. The orientations of the wild-type and mutant MSP-HEWL inserts in the pRG4 constructs is indicated. (The position of the ATG initiation codon in the avian HEWL prosequence is represented ATG).

The presence and orientation of the MSP-HEWL construct in pRG4 was then ascertained by restriction mapping plasmids from transformants, using BamHI and EcoRI. Agarose gel electrophoresis data indicated that the orientation of the construct with respect to both the BamHI and EcoRI sites of the polylinker of pRG4, and to the BamHI and EcoRI sites in the MSP HEWL insert, was as shown for pRG4;MSP-proHEWL Loop- C94T and pRG4;MSP proHEWL in Fig. 4 below.

### Expression of HEWL in N. crassa

Two strains of N. crassa, one defective in pyr4 gene function and the other defective in both pyr4 and in am132 gene function, were transformed with the pRG4;Pro-HEWL plasmids.

Transformants were obtained from both strains and also with the wild type and the mutant plasmids. However, expression of HEWL by these transformants could not be detected either by protein gel electrophoresis or immunological means. This could be attributed to either a failure of homologous recombination to successfuly incorporate the construct into the genome without peturbation of the sequence or to a defect which limited the transcriptional efficiency of the promoter.

Using inverse PCR, the native avian pro sequence to HEWL was replaced with the signal sequence for a protein (aAG, alpha-amyloglucosidase) from the fungus Aspergillus niger.

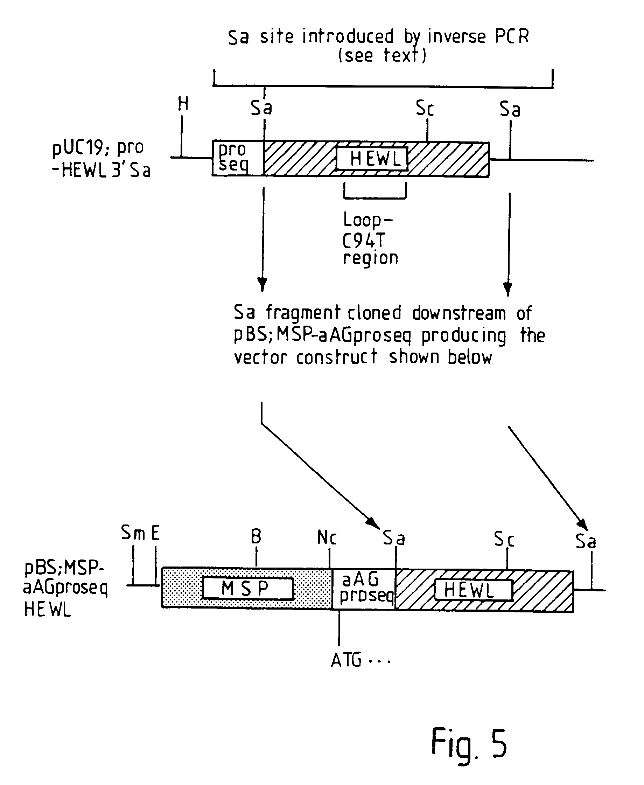

This is shown schematically in Figure 5, which represents the mechanism used to replace the native avian prosequence to HEWL with the signal sequence of a fungal protein (aAGproseq). The position of the initiation codon is shown by ATG. The Sa fragment of pUC19; proHEWL3' Sa was inserted downstream of the pBS; MSP-aAGproseq site, to produce the construct pBS; MSP-aAGproseq HEWL.

Therefore, if N. crassa could not in some way deal with the avian pro-sequence, this would be expected to be alleviated by the fungal sequence. However, this did not overcome the problem.

Further studies with the aim of developing a regulatable expression vector system for use in N. crassa showed that the MSP promoter was only capable of low level constitutive expression. Therefore, it would be unlikely for HEWL to be detectable. Therefore, as final success with this expression system was going to take more time than was available it was decided to switch to an expression system which had been shown to be capable of producing HEWL in high yield, albeit in inclusion bodies and with a Met⁻¹ residue. These studies are reported in Example 2.

### Example 2

### Cloning and Expression of a wild-type and a mutant HEWL gene in E. coli

It has been pointed out already that wild-type HEWL has been cloned into a plasmid expression vector and expressed in E. coli with high efficiency (Miki et al. 1987 Protein Engineering 1, 327-332). Therefore, on acquiring the plasmid vector, pKP1500, and the E. coli host cell strain used in this work, KP3998 (both described by Miki et al., cited above), it was possible to start to recreate HEWL expression vectors.

### Production of a wild type HEWL expression cassette

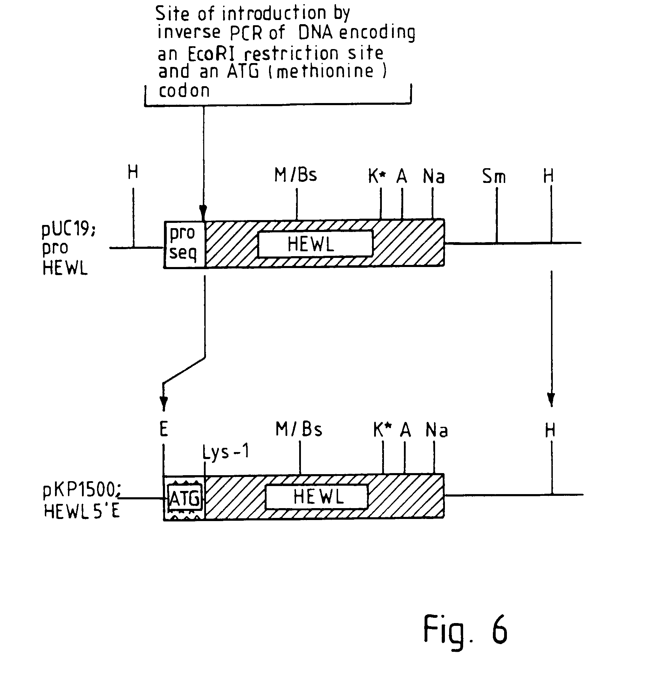

The first step undertaken was to introduce an EcoRI restriction site, incorporating a methionine (ATG) start codon 5' and immediately upstream to the amino acid codon for lysine 1 (Lys-1). This Eco RI site and ATG start codon are indicated in Figure 6 by the arrow at the end of the "proseq" region.

The mutation was achieved by means of the inverse PCR procedure using a wild type HEWL gene construct in pUC19 vector, pUC19;proHEWL, and the 28-mer mutagenic primer T007174. After Klenow and T4 Polynucleotide kinase enzyme treatment, the linear inverse PCR DNA product was religated and transformed into TG1 cells.

This resulted in the production of a pUC19 based plasmid which retained the restriction enzyme sites shown in Fig. 6. Transformants were identified that not only released a HindIII fragment similar in size to the wild-type HindIII fragment recovered from pUC19:proHEWL but also released a fragment that was marginally smaller when the plasmid DNA was digested with HindIII/EcoRI, which confirmed that the EcoRI site had indeed been successfully introduced at the correct location.

Plasmid DNA was therefore digested with EcoRI and HindIII to yield a sticky-ended HEWL fragment. This enabled the correct orientation of the fragment with respect to the vector and successful transformation was achieved when the fragment was ligated into HindIII/EcoRI cut and purified pKP1500 vector. The ligation mix was used to transform competent KP3998 cells yielding the wild-type expression vector shown in Fig. 6.

A number of transformants screened with EcoRI and HindIII showed release from their plasmids of the HEWL fragment. At this time sufficient cell stocks of the transformants were frozen with DMSO at -196°C to subsequently allow the production of cell paste from fermenters. This avoids the possibility of plasmid loss or recombination events taking place in KP3998 (a rec+ strain) due to unnecessary cycles of sub-culturing. For the same reasons stock plates for this system were not maintained at 4°C.

The wild type HEWL in pKP1500 was therefore believed to retain the restriction sites shown in Fig. 6.

### Production of a mutant HEWL expression cassette

The production of an expression vector for the loop-mutant lysozyme was considered at the outset to be a relatively straightforward task. It was recognized that there was no 3' HindIII site in the mutant lysozyme clone which would allow interchange of a MamI/BsaBI - HindIII fragment between it and the wild-type clone (compare upper diagram in Fig. 6 with diagram no. 4 in Fig. 7) but anticipated that the mutated region in the loop- lysozyme clone could be removed as either a MamI-Kpn1 or a BsaBI-Kpn1 fragment, which could be ligated into the corresponding region of the wild type gene which, in pKP1500, already possessed the EcoRI site and methionine (ATG) codon adjacent to Lys 1, as depicted in Fig. 6.

It was observed that the mutant lysozyme gene would cut with KpnI and, with restriction with either MamI or BsaBI in addition to KpnI, would release a fragment from the plasmid of a size very similar to that which would be expected for the mutant HEWL. On the other hand the plasmid containing the wild-type HEWL gene, which had undergone inverse PCR to introduce the 5' EcoRI site and Met⁻¹ codon, failed to show any restriction with KpnI. It was therefore impossible to achieve the objective. It was equally important to establish the cause of the problem, to be sure that the inverse PCR had not introduced a random (undesired) mutation.

This anomalous behaviour between the two plasmids in their restriction with KpnI was resolved. The loop mutant lysozyme gene cut with KpnI because it retained a KpnI site from the pUC19 vector multiple cloning site, that had not been eliminated in former manipulations of this DNA.

But this did not account for the loss of the KpnI site in the coding region of the wild-type gene (or possibly the mutant too), which was predicted to be present from authentic gene sequences in a database.

The available on-site sequence data for the construct at this region was equivocal for just one base. The automatic DNA sequencer was able to limit its call for the base to a Y (i.e. C or T) but it could not differentiate one from the other, unequivocally. Therefore, the possibility existed that there was a silent mutation in the sequence. This required further examination with KpnI, of the sub-clones of the gene which had been produced since the original clone was received into the laboratory.

The present inventors followed this failure to cut with KpnI all the way back to the original stab culture of pTK2;proHEWL, and so it could only be concluded that the gene had been received into the laboratory with a mutation at the KpnI site. Fortunately, the mutation is at a position that results in a silent mutation. Therefore, because this would not affect the amino acid sequence of the protein it was left until much later to confirm with new sequence data this conclusion.

An alternative strategy was therefore adopted. Instead of KpnI restriction, it was shown that both clones possessed the NaeI site close to the stop codon for HEWL (see Fig. 7, diagram 1). Therefore, transfer of the BsaB1-NaeI fragment by blunt-ended ligation was attempted. This worked with high efficiency but, despite the fact that enough transformants were analysed to be able to detect a ligation occurring in the correct orientation at a frequency of less than 0.04, no transformant with the desired characteristics could be detected.

The inventors therefore resorted to using BsaBI with AatII, which would produce a blunt-sticky ligation and so help to orientate the fragment in the desired way. Again this worked with high effiency but regeneration of the respective restriction enzyme sites, (which would be a prerequisite to any further examination of the transformants), could not be demonstrated.

A series of test and control digestions, using all possible combinations of the separate components in the restriction enzyme reaction mixtures revealed there was some low, non-specific nuclease contamination in these two, rather infrequently used, enzymes. This produced some smearing of the plasmid DNA in agarose gel electrophoresis. This would probably have been sufficient to "nibble" dNTP bases from the ends of the restricted DNA and prevent essential regeneration of the restriction enzyme sites. It was useful that the inventors could not detect any such evidence for non-specific nuclease contamination of the plasmid DNA preparations.

At this stage the present inventors found themselves highly constrained by the lack of further restriction enzyme sites that would be specific for their requirements. They therefore adopted a less conventional approach to resolving their problems, which nevertheless was successful. One advantage in this was that the inventors worked on the HEWL sequence outside the protein coding region.

The first manipulation undertaken with the plasmid was to eliminate the EcoRI site which remained in the plasmid 3' to the end of the coding sequence (see Fig. 7, diagram 5) as part of the pUC19 multiple cloning site. This was accomplished by restricting the pUC19;proHEWLC94TLoop-3'Sa plasmid with EcoRI, which, as expected, linearized the construct. Then a synthetic linker sequence was ligated into the junction, thereby recircularising the plasmid. In so doing, by judicious design of the linker, the EcoRI site was-replaced with a HindIII site as shown below. (The lower case bases show the synthetic linker sequence.)

This procedure worked very well. Of 12 transformants selected for examination, 9 released a HindIII fragment of an appropriate size, as revealed by agarose gel electrophoresis restriction digest analysis. Those transformants that did not release a fragment with HindIII did, on the other hand, linearize with EcoRI, as expected.

Vice versa, the HindIII positive clones did not linearize with EcoRI, the result to be expected from substitution of the EcoRI site with the HindIII site, as shown in diagram 5 of Fig. 7.

Having achieved the introduction of a HindIII site 3' to the stop codon of the HEWL sequence it became feasible to attempt exchange the BsaBI - HindIII fragment of the mutant gene with that of the wild type. It will be recognized that the HindIII sites in the two clones were not in exactly the same position in the sequence at this time. However, falling outside the protein coding region this difference in the position of the HindIII sites would not have been expected to influence the subsequent expression of the mutant protein when transferred into pKP1500.

This was another blunt-sticky ligation, and therefore would not have been expected to be particularly difficult, but it failed to produce any transformants that would recut with BsaBI. As discussed previously, regeneration of the BsaBI restriction enzyme site would be essential in indicating that the fragment had been interchanged in-frame, so that the correct DNA codons would be transcribed into expressed protein. Based on earlier problems experienced with BsaBI and its isoschizomer MamI, in attempting to obtain fragments that would religate and satisfactorily reform their restriction sites, it was decided to abandon use of this enzyme. Instead it was decided once again to use inverse PCR to introduce the 5' site for EcoRI into the mutant HEWL sequence.

Earlier, several attempts were made to introduce, by inverse PCR, the 5' EcoRI site and Met⁻¹ codon adjacent to Lys-1 into the mutant gene sequence, by using remaining stocks of the oligonucleotides which had been successfully used with the wild type gene. For some reason these oligonucleotides would not produce a clearly defined reaction product, although the reactions were titrated with Mg²⁺ and the DNA template, although observed to be 'clean' when studied by agarose gel electrophoresis, was also repurified by phenol-chloroform extractions etc.

Rather than expend time on resolving the cause for failure it was more efficient to resynthesize the oligonucleotides. Two new primers for this inverse PCR, only marginally different to the former, were therefore designed and synthesized. The forward mutagenic primer, which would introduce the EcoRI site and a Met⁻¹ start codon, was the 30-mer shown below. The melting temperature was calculated to be 68°C. The 20-mer shown below had a corresponding melting temperature of 66°C and constituted the reverse primer.

Our inverse PCR procedures normally use an annealing temperature of 55°C. For the above oligonucleotides this was considered to be very low in relation to the calculated melting temperatures of the oligonucleotides. Because it was desired to limit the occurrence of false priming events leading to PCR products of diverse sizes, the first inverse PCR used an annealing temperature of 62°C. This was either too high or the reaction failed due to insufficient primers or dNTPs. However the ensuing reaction, performed using an annealing temperature of 58°C was very satisfactory yielding a single band of PCR product when examined by agarose gel electrophoresis. When compared with HindIII-cut lambda DNA markers the band was observed to be approximately 3kbp, as would be expected for this plasmid construct.

The PCR product was therefore treated with Klenow and T4 polynucleotide kinase enzymes prior to purification by gel electrophoresis, and religation. The religated product was used to transform competent Sure cells (a rec-strain). Confirmatory restriction digests with HindIII, EcoRI and EcoRI/HindIII were performed on plasmids isolated from transformants. These showed that the PCR had worked satisfactorily.

Agarose gel electrophoresis revealed that of 8 transformants examined, 6 released a fragment of approximately 500bp with HindIII. These 6 plasmids also linearized with EcoRI. On the other hand, combined digestion with EcoRI and HindIII released a fragment smaller than the fragment of approximately 500bp released by HindIII alone. This was the expected result confirming that the EcoRI site had been introduced between the HindIII sites. It allowed progression to the preparation of EcoRI-HindIII fragments from several transformants (by agarose gel electroelution) and to ligation into the vector pKP1500. This had been prepared in a similar manner, after digestion with the same enzymes.

The ligation proceeded according to plan and the transformation procedure was again performed with Sure cells. Of the 24 transformants screened, 22 contained an insert of the appropriate size. From these, 4 were selected at random for further study. In particular, it was essential to know that there had been no religation of an incompletely cut vector, although every effort and precaution had been taken in the preparation of the enzyme restricted vector fragment to prevent this occurring.

The discriminatory tests which confirmed that we had achieved the expected result resided in the differences between the mutant and wild type clones at their 3' ends, that had earlier caused difficulties. For example, both Sall and KpnI sites were to be expected in our transformants. Furthermore, to be consistent with our understanding of the earlier manipulations that had been carried out with the mutant gene in this region (see Fig. 7) we expected that EcoRI/SalI digestions would yield fragments that would run marginally ahead of EcoRI/KpnI fragments. This was confirmed empirically. In addition, we later substantiated these deductions and observations directly with sequence data.

Having ensured that the correct construct had been made, KP3998 cells were transformed with plasmid from the 4 clones described above. This enabled frozen DMSO stocks of transformants to be made. These would be suitable not only for the production of cell paste in fermentors but also for the preparation of plasmid DNA, that would be subjected to unequivocal final confirmatory DNA sequencing.

### DNA Sequence Data on Mutant HEWL in pKP1500

In order to sequence the gene and to define the junctions between the gene and the pKP1500 vector, four oligonucleotides were synthesized. The sizes and sequences of these oligonucleotide primers are given below, together with an indication of their location and the direction of sequencing of the gene.
- G6875: Sequences along the non-coding strand from approximately nt+40 towards 5' junction of the gene with vector.
- G6904: Sequences 5' to 3' on coding strand from approximately 30nt upstream of the loop deletion.
- G6857: Sequences 5' to 3' on coding strand from approximately 15nt downstream of the loop deletion through the stop codon to 3' junction with vector.
- G6869: Sequences 5' to 3' on coding strand from the start of the gene sequence to sequence the region lying between G6875 and G6904.

The DNA from two different clones of pKP1500;HEWLC94TLoop- was sequenced using the double-stranded DNA sequencing protocol recommended by Dupont for the Genesis 2000 Automatic Sequencer.

Sequence data were obtained for the entire gene sequence and the immediate flanking regions comprising vector and remains of the pUC19 multiple cloning site. The important observations were as follows:

At the 5' junction of the gene with vector the sequence read:

The underlined codon is that of Lys-1 and the lower case codon immediately before is that of the introduced Met⁻¹. The underlined sequence immediately upstream is that of the introduced EcoRI restriction site.

The sequence in the region of the loop deletion was as given below in a) (Seq. ID No. 11), indicating satisfactory engineering. The sequence b) (Seq. ID No. 12) also shows correct mutagenesis of codon 94 from Cys to Thr. The ensuing sequence in c), (Seq. ID No. 13), confirms that in this DNA the KpnI site, at what would be codons 117-118 in native HEWL, is lost by a T to C mutation. This confirms the conclusions drawn earlier concerning the apparently anomalous restriction of the plasmids with KpnI.

Beyond the HEWL stop codon, the 3' flank to the gene had the following DNA sequence, shown below (Seq. ID No. 14). This data again confirms the deductions made earlier using restriction enzymes which identified the presence and orientation of particular sites in this region of the plasmid.

The stop codon (1) is shown in lower case. The SalI site (2) and the KpnI site (3) are underlined. The HindIII site (4) inserted with a linker is shown in lower case underlined. The position of the EcoRI site (5), destroyed by the introduction of the HindIII site 5' adjacent to it, is shown double underlined.

### Expression of the wild-type and mutant HEWL proteins in culture

The HEWL genes inserted into the pKP1500 vector and transformed into KP3998 cells were expressed in fermenter cultures. For this purpose small overnight cultures were prepared from frozen stocks of cells using a broth similar to that described by Miki et al. (1987 cited previously). The overnight cultures were then used to prepare a culture for inoculating the fermentors. At this stage the cultures were grown at 28°C. The subsequent conditions required for expression of the wild-type and the mutant gene were obtained from experiments in our own laboratory. When attempting to reproduce the conditions only poorly described by Miki et al. (1987), neither satisfactory cell growth nor protein expression were obtained.

### Fermentation conditions

It was found that high aeration rates are essential for good cell yields and protein expression. As a result the present inventors routinely use the maximum the fermenter vessels will provide (201 min⁻¹) and add antifoam to prevent undue frothing. In addition much more glycerol is added, as an extra carbon source, than that quoted by Miki et al. (1987). Finally, when the cultures are induced with IPTG, additional antibiotic is added to maintain cell selection pressure. Culture temperature at this stage is 40°C.

It has also been found that the cultures cannot be incubated indefinitely. After about 15 hours post induction specific protein yield falls dramatically, although the broth continues to increase in OD₅₅₀, albeit rather slowly. This is attributed to loss of antibiotic and therefore selection pressure to maintain the plasmid. At this time nutrients also become limiting, but pH does not fall appreciably.

Using these refinements it was possible to obtain about 150g of cell paste from each 10 litre fermenter. In the case of the wild-type gene, the proportion of cell proteins accounted for by HEWL was approximately 17% when assessed by SDS PAGE (laser densitometry). The mutant protein was expressed equally vigorously yielding comparable amounts of cell paste and a proportion of 25% of total cell proteins (see Table 1).

The difference between the wild-type and mutant genes in these proportions is not considered significant, based on the particular fermentations from which these data were obtained. However, the data are remarkably similar to the values quoted by Miki et al. (1987). The common feature is that cultures were induced at the same cell density in both studies (i.e. 0.6 O.D. at 660nm).

All clones of HEWL that have been expressed in fermenters appear to require at least 4 hours of IPTG induction before significant quantities of HEWL are observed in the cell paste. Then, between 6 hours and 12 hours post-induction the above quoted proportions of cell proteins occurring as HEWL do not seem to vary significantly, although cell yield is increasing. However, after 15h there is a significant rapid decline in HEWL yield. Since culture for more than 12h results in only limited further cell growth, production was routinely performed overnight for 12h to maximize yields.

### Example 3

### The biochemical isolation of HEWL

The isolation and purification of HEWL to a point that it appears homogenous in SDS PAGE was accomplished relatively easily.

Briefly, cells were washed with isotonic phosphate buffer and suspended in the same buffer containing PMSF and EDTA, before French pressing three times. The inclusion bodies, containing HEWL, were harvested by centrifugation and washed. Inclusion bodies were then purified by suspending them in buffered 2M area after which they were collected by centrifugation at 10,000g. Lysozyme was then purified from the pellet by solubilisation in TrisHCl buffered 8M urea, pH 8.6, containing 2-mercaptoethanol, equivalent to 1 x 10⁻⁵ mg⁻¹ protein. At this stage the protein concentration was 10-20 mg ml⁻¹ and the solution was incubated at 37°C for 1 hour.

**Table 1**

| The purification of recombinant wild-type and mutant lysozymes from E. coli. | | | | |
|---|---|---|---|---|
| | Lysozymes as % of total proteins in sample | | Purification factor | |
| Process stage | wild-type | mutant | wild-type | mutant |
| E. coli cells | 17 (8g cell) | 25 (15g cells) | | |
| Inclusion bodies | 78 | 94 | 4.6 | 3.7 |
| Urea washed inclusion bodies | 89 | 96 | 5.2 | 3.9 |
| Sephacryl S 100 eluate | 98 | 99 | 5.8 | 4.0 |

The wild type and mutants each produced approx. 50mg protein in inclusion bodies per 8g cells processed. The urea washed inclusion bodies retained approx. 80% of the total protein and 75% of this material was recovered in the Sephacryl S 100 eluates, giving a yield of 30mg lysozyme.

The percentage of lysozyme in the different process fractions was determined by laser densitometry of proteins in SDS PAGE.

The 10,000g supernatant was then subjected to Sephacryl HR S-100 gel filration. After collection of the second main protein peak, 2-mercaptoethanol was added to the former concentration and the solution incubated for 1 hour at 37°C. After acidification to pH 4 with glacial acetic acid the material was passed through Sephadex G-25 equilibrated with 0.1M acetic acid. The eluate revealed only 1 band on SDS PAGE and about 30 mg of protein were obtained from 8g wet weight of cells.

### Protein refolding and biological activity

The wild-type HEWL was refolded at 37°C under anaerobic conditions, using nitrogen, in 0.1M Tris acetate buffer pH 8.0, which also contained 1mm reduced glutathione and 0.5mM oxidised glutathione and 1mm EDTA. The protein concentration was 10 x 10⁻⁶g ml⁻¹ and after 2 hours it was found that nearly two-thirds of the protein had refolded. This was assessed by SDS gel electrophoresis under nonreducing conditions. By comparison of the material with native HEWL it was possible to determine from the electrophoretic mobility the size and abundance of the molecular species that had refolded in the same manner as native HEWL. The progress of refolding is given in Table 2A. It was also observed that the recombinant Met⁻¹ protein refolded at about 50% of the velocity of native HEWL treated in the same way. However, it was observed that after purification of refolded recombinant material from non-refolded material the refolded lysozyme possessed the same protein specific activity as the native substance isolated from eggs.

Therefore, it appears that the presence of a Met⁻¹ residue at the N terminal end of the protein adjacent to Lys-1 has little effect on biological activity, but it may significantly influence the mechanisms by which the protein refolds.

The mutant HEWL exhibited identical properties to the combinant wild-type during isolation and purification. The content of free -SH groups in the mutant and the recombinant wild-type were compared to the native protein and found to be in excellent agreement. The native protein gave an expected value of 8. The recombinant wild-type gave values between 8 & 10 (9 expected). The mutant gave values of 7, which agrees with the expected value. Furthermore in reducing SDS gel electrophoresis it was possible to detect the smaller size of the protein when compared with the native or the recombinant wild-type lysozyme.

The enzyme activity of the mutant and wild type lysozyme molecules was assayed using standard procedures, as described by Fischer et al. (1992, Archives Biochem. Biophys. 298, 361-364). Similarly, the pH activity profile for the molecules was investigated using the assay method of Fischer et al., but under various pH conditions (66mM citrate phosphate for pH 3.0-4.5, 66mM sodium phosphate in the range 5.0-7.5, 66mM Tris for the range 8.0-9.0, and 66mM sodium borate in the range 9.5-10.0). M. lysodeikticus cells were used at 0.15 mgs/ml.

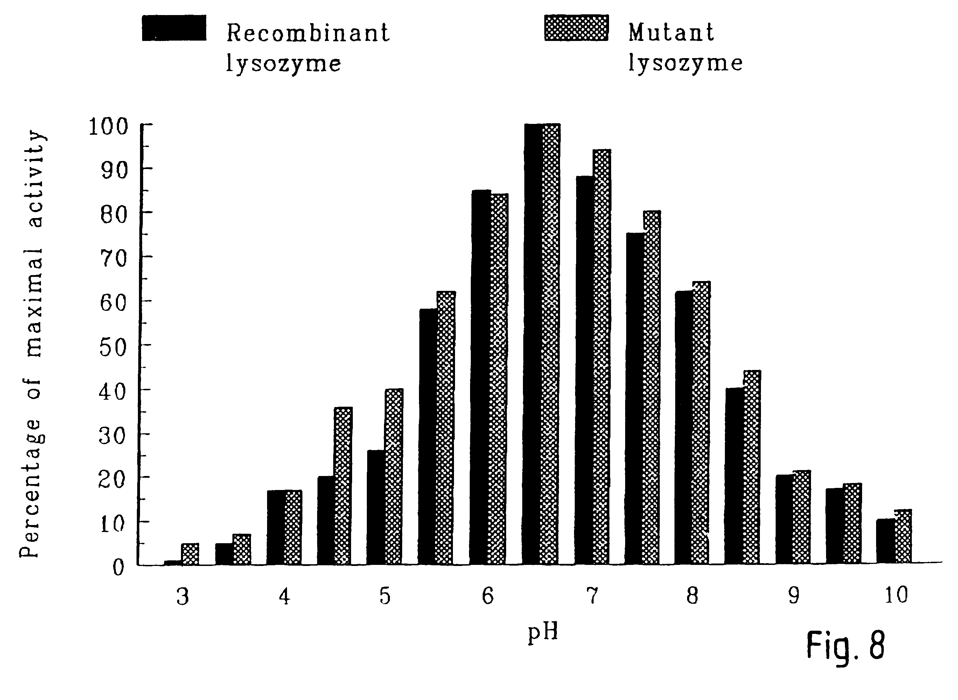

The activity profile for recombinant wild type (solid bar) and mutant (hatched bar) lysozymes is shown in Figure 8, which is a bar chart showing percent of maximal activity over a range of pH values, from 3-10. The chart shows that the wild-type and mutant enzymes have substantially similar profiles and share the same pH optimum.

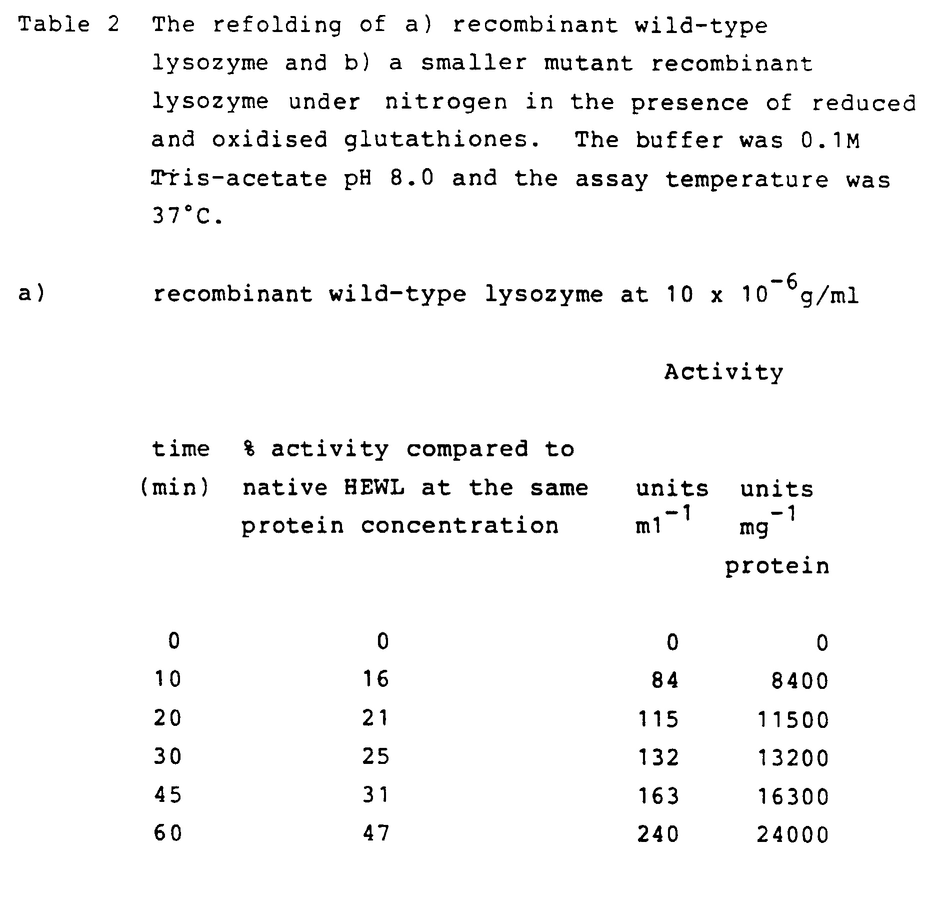

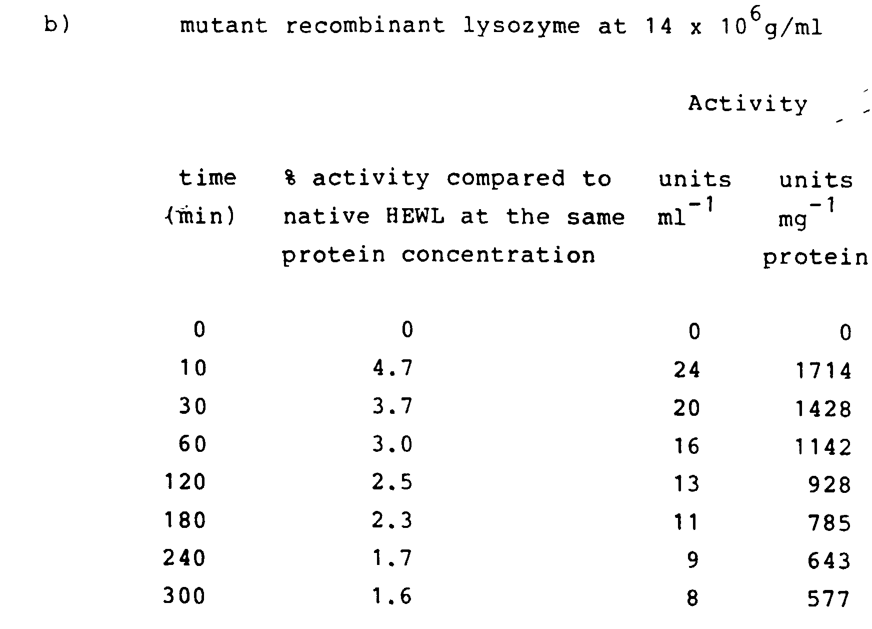

It might be that the factors which account for the slower velocity of refolding seen with the Met⁻¹ wild type recombinant lysozyme are much more important with the smaller mutant gene, which also possesses a Met⁻¹ residue. Nevertheless, a consistent and repeatable observation has been that some biological acitivity is found when mutant material is examined for activity after various periods of time in the refolding procedure. The activity is not high but it is consistently above those values considered as "background" (Table 2b).

Unfortunately, the activity also declines with the progression of refolding (Table 2b). The biochemical reasons for this have yet to be established. It appears as though the material is conformationally moving from one molecular state in which it has activity to some other state where this activity is lost.

Studies indicate that the action of lysozyme on the peptidoglycan of spores is limited by the permeability of the enzyme through the spore coat. A lysozyme that causes undamaged spores to germinate and become susceptible to mild heat treatment would revolutionise the ways in which foods can be stored. Lysozyme can cause the germination of bacterial spores whose spore coats have been weakened by heat or chemical treatment and of spores that are genetically defective. Together with measurements that indicate the size of the pores and the size of hen lysozyme are similar this suggests that a smaller lysozyme may be able to penetrate intact bacterial spores. Bacterial spore coats are also known to allow basic molecules to permeate more readily than neutral or acidic ones. Hen lysozyme is both the smallest and most basic lysozyme and loop removed forms may be suitable for neutralizing bacterial spores.

### SEQUENCE LISTING

(I) GENERAL INFORMATION:
   (i) APPLICANT:
      (A) NAME: Unilever PLC
      (B) STREET: Unilever House, Blackfriars
      (C) CITY: London
      (E) COUNTRY: United Kingdom
      (F) POSTAL CODE (ZIP): EC4P 4BQ
      (G) TELEPHONE: (071) 822 5252
      (H) TELEFAX: (071) 822 5954

      (A) NAME: Unilever N.V.
      (B) STREET: Weena 455
      (C) CITY: Rotterdam
      (E) COUNTRY: Netherlands
      (F) POSTAL CODE (ZIP): 3013 AL
   (ii) TITLE OF INVENTION: Alteration of Polypeptides
   (iii) NUMBER OF SEQUENCES: 15
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentln Release #1.0, Version #1.25 (EPO)
## Claims

1. A biologically functional altered lysozyme molecule comprising fewer amino acid residues and being reduced in size compared with an unaltered lysozyme molecule, wherein the altered lysozyme lacks at least part of the loop region of amino acid residues Cysteine 64 to Cysteine 80 of the unaltered molecule.

2. An altered lysozyme molecule according to claim 1, wherein amino acid residues between Cysteine 64 and Cysteine 80 are replaced with the amino acid sequence asparagine-glycine-serine-asparagine.

3. An altered lysozyme molecule according to claim 1 or 2, comprising an altered hen egg white lysozyme having a threonine residue at position 94.

4. An altered polypeptide according to any one of claims 1, 2 or 3, having greater ability to penetrate bacterial spore or cell envelopes compared to the unaltered polypeptide.

5. A method of producing a biologically functional altered lysozyme molecule comprising fewer amino acids and being reduced in size compared with an unaltered lysozyme molecule, comprising: altering the nucleic acid sequence encoding a biologically functional lysozyme molecule such that the latter lacks at least part of the loop region of amino acid residues Cysteine 64 to Cysteine 80 present in the unaltered lysozyme molecule; and causing expression of the altered nucleotide sequence so as to produce the biologically functional altered lysozyme.

6. A method according to claim 5, wherein the alteration is effected by polymerase chain reaction.

## Patentansprüche

1. Biologisches aktives modifiziertes Lysozym-Molekül, umfassend weniger Aminosäurereste und mit im Vergleich zu einem unmodifizierten Lysozym-Molekül reduzierter Größe, wobei dem modifizierten Lysozym zumindest ein Teil der Schleifenregion der Aminosäurereste Cystein 64 bis Cystein 80 des unmodifizierten Moleküls fehlt.

2. Modifiziertes Lysozym-Molekül nach Anspruch 1, wobei die Aminosäurereste zwischen Cystein 64 und Cystein 80 durch die Aminosäuresequenz Asparagin-Glycin-Serin-Asparagin ersetzt sind.

3. Modifiziertes Lysozym-Molekül nach Anspruch 1 oder 2, umfassend ein modifiziertes Hühnereiweiß-Lysozym mit einem Threoninrest an Position 94.

4. Modifiziertes Polypeptid nach einem der Ansprüche 1, 2 oder 3, welches im Vergleich zum unmodifizierten Polypeptid eine höhere Fähigkeit zur Penetration bakterieller Sporen oder Zellhüllen aufweist.

5. Verfahren zur Herstellung eines biologisch aktiven modifizierten Lysozym-Moleküls, umfassend weniger Aminosäuren und mit im Vergleich zu einem unmodifizierten Lysozym-Molekül reduzierter Größe, bei dem eine ein biologisch aktives Lysozym-Molekül codierende Nukleinsäuresequenz dergestalt modifiziert wird, daß letzterem zumindest ein Teil der im unmodifizierten Lysozym-Molekül vorliegenden Schleifenregion der Aminosäurereste Cystein 64 bis Cystein 80 fehlt, und die Expression der modifizierten Nukleotidsequenz veranlaßt wird, so daß das biologisch aktive modifizierte Lysozym produziert wird.

6. Verfahren nach Anspruch 5, wobei die Modifizierung durch Polvmerasekettenreaktion bewirkt wird.

## Revendications

1. Molécule de lysozyme biologiquement fonctionnellement modifiée comprenant un moindre nombre d'acides aminés et de taille réduite par comparaison avec celle de lysozyme non modifié, le lysozyme modifié étant dépourvu d'au moins une partie de la région de boucle des résidus acides aminés Cystéine 64 à Cystéine 80 de la molécule non modifiée.

2. Molécule de lysozyme modifiée selon la revendication 1, dans laquelle les résidus acides aminés entre la Cystéine 64 et la Cystéine 80 sont remplacés par la séquence d'acides aminés asparagine-glycine-sérine-asparagine.

3. Molécule de lysozyme modifiée selon la revendication 1 ou 2,comprenant un lysozyme de blanc d'oeuf de poule modifié ayant un résidu thréonine en position 94.

4. Polypeptide modifié selon l'une quelconque des revendications 1, 2 ou 3, ayant une plus grande aptitude à pénétrer les spores bactériennes ou les enveloppes bactériennes par comparaison avec le polypeptide non modifié.

5. Procédé de production d'une molécule de lysozyme biologiquement fonctionnellement modifiée comprenant un moindre nombre d'acides aminés et étant de taille réduite par comparaison avec une molécule de lysozyme non modifiée, dans lequel : on modifie la séquence d'acides nucléiques codant pour une molécule de lysozyme biologiquement fonctionnelle de manière que cette dernière est dépourvue d'au moins une partie de la région de la boucle d'acides aminés Cystéine 64 à Cystéine 80 présente dans la molécule de lysozyme non modifiée ; et on provoque l'expression de la séquence de nucléotides modifiés de manière à produire le lysozyme biologiquement fonctionnellement modifié.

6. Procédé selon la revendication 5, dans lequel la modification est effectuée par une réaction en chaîne de polymérase.


(2) INFORMATION FOR SEQ ID NO: 1:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 1:
   GAGCGTGAAC ACGGCGAAGA AGAT 24
(2) INFORMATION FOR SEQ ID NO: 2:
   (i) SEQUENCE CHARACTERISTICS:
      (A) LENGTH : 48 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 2:
   ACAGCCGCTG GTGGTGCAAC GGTAGCAACT GCTCAGCCCT GCTGAGCT 48
(2) INFORMATION FOR SEQ ID NO: 3:
   (i) SEQUENCE CHARACTERISTICS:
      (A) LENGTH: 28 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 3:
   AGCTCGAATT AAGCTTAATT CACTGGCC 28
(2) INFORMATION FOR SEQ ID NO: 4:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEO IV NO: 4:
   GCTGCTCTGG GAATTCTATG AAAGTCTTTG 30
(2) INFORMATION FOR SEQ ID NO: 5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 5:
   GGGGCAGGAA GCAAAGCACC 20
(2) INFORMATION FOR SEQ ID NO: 6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 6:
   CCGATAGTTA TCAAGTCCGT G 21
(2) INFORMATION FOR SEQ ID NO: 7:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 7:
   CGATGGGAGT ACCGACTACG G 21
(2) INFORMATION FOR SEQ ID NO: 8:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 8:
   GAGCTCAGAC ATAACAGCGA G 21
(2) INFORMATION FOR SEQ ID NO: 9:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 9:
   GTCTTTGGAC GATGTGAGCT GG 22
(2) INFORMATION FOR SEQ ID NO: 10:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 10:
   TCACACAGGA AACAGAATTC TATGAAAGTC 30
(2) INFORMATION FOR SEQ ID NO: 11:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 11:
   GGTGGTTGCA ACGGTAGCAA CTGCTCA 27
(2) INFORMATION FOR SEQ ID NO: 12:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 12:
   AGCGTGAACA CCGCGAAGAA G 21
(2) INFORMATION FOR SEQ ID NO: 13:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 13:
   CGCTGCAAGG GCACCGACGT C 21
(2) INFORMATION FOR SEQ ID NO: 14:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 14:
   CTGTGAGGAG CTGCCGCGCC CCCGTCGACG GGGGTACCG 39
(2) INFORMATION FOR SEQ ID NO: 15:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 15:
   GGCGGTTTTG TCGGTTCGAA TTAAGCTCGA 30